Europäisches Patentamt

European Patent Office

Office européen des brevets

(11) Veröffentlichungsnummer: **0 336 077**
**A1**

## (12) EUROPÄISCHE PATENTANMELDUNG

(21) Anmeldenummer: 89102468.9

(22) Anmeldetag: 14.02.89

(51) Int. Cl.⁴: **C07D 301/32 , C07D 303/08**

(30) Priorität: 06.04.88 DE 3811524

(43) Veröffentlichungstag der Anmeldung:
**11.10.89 Patentblatt 89/41**

(84) Benannte Vertragsstaaten:
**AT BE CH DE ES FR GB GR IT LI LU NL SE**

(71) Anmelder: **DEUTSCHE SOLVAY-WERKE GMBH**
**Langhansstrasse 6 Postfach 11 02 70**
**D-5650 Solingen 11(DE)**

(72) Erfinder: **Jakobson, Gerald, Dr.Dipl.-Chem.**
**Willinger Weg 21**
**D-4134 Rheinberg 2(DE)**
Erfinder: **Siemanowski, Werner, Dr.**
**Dipl.-Chem.**
**Am Annaberg 18**
**D-4134 Rheinberg 1(DE)**

(74) Vertreter: **Seiler, Siegfried**
**c/o DEUTSCHE SOLVAY-WERKE GmbH**
**Langhansstrasse 6 Postfach 11 02 70**
**D-5650 Solingen 11(DE)**

(54) Verfahren und Vorrichtung zur Herstellung von Reinst-Epichlorhydrin.

(57) Die vorliegende Erfindung betrifft ein Verfahren und eine Vorrichtung zur Herstellung von Reinst-Epichlorhydrin aus technische Verunreinigungen enthaltendem Epichlorhydrin durch Destillation in einer Kolonne, die mit einer am Boden oder in der Nähe des Bodens der Kolonne angeordneten Verdampfungs- oder Erhitzungsvorrichtung ausgestattet ist. Das technische Epichlorhydrin wird dabei einer fraktionierten Destillation unterworfen, wobei das technische Epichlorhydrin in einem Abstand von der Verdampfungs- oder Erhitzungsvorrichtung eingeleitet wird, der größer als 1/10, vorzugsweise größer als 1/4, der gesamten Kolonne beträgt und das Reinst-Epichlorhydrin in einem Abstand von der Verdampfungs- oder Erhitzungsvorrichtung abgezogen wird, der größer als ein Drittel, vorzugsweise größer als die Hälfte der gesamten Kolonne ist. Die Verunreinigungen mit leichtsiedenden Bestandteilen und mit schwersiedenden Bestandteilen werden am Kopf bzw. am Fuß der Kolonne kontinuierlich abgezogen.

EP 0 336 077 A1

## Verfahren und Vorrichtung zur Herstellung von Reinst-Epichlorhydrin

Die vorliegende Erfindung betrifft ein Verfahren und eine Vorrichtung zur Herstellung von Reinst-Epichlorhydrin aus technische Verunreinigungen enthaltenden Epichlorhydrin durch Destillation in einer Kolonne, die mit einer am Boden oder in der Nähe des Bodens der Kolonne angeordneten Verdampfungs- oder Erhitzungsvorrichtung ausgestattet ist, wobei durch bestimmte Verfahrensführungen und/oder Vorrichtungsmerkmale bzw. Vorrichtungskombinationen die Herstellung von Reinst-Epichlorhydrin verbessert wird.

Aus der DE-AS 1 210 777 ist bereits ein Verfahren zur Herstellung von Epichlorhydrin durch Dehydrochlorierung von 1,2-Dichlorpropanol-(3) mit alkalischen Mitteln in wäßrigem Medium bei erhöhter Temperatur bekommt. Aus dem Beispiel 1, Absatz 2 geht hervor, daß das Rohepichlorhydrin über eine 20-Bödenkolonne rektifiziert und ein Reinprodukt von Kp 115 bis 116 °C neben nicht umgesetzten Dichlorpropanol erhalten wird. Das so erhaltene Epichlorhydrin weist jedoch auch nach der Rektifikation störende Verunreinigungen auf, u.a. in Form von Halogenkohlenwasserstoffen und dgl..

Ziel und Aufgabe der vorliegenden Erfindung war es, diese Nachteile zu beseitigen und ein Verfahren und eine Vorrichtung zu finden, in dem bzw. der Reinst-Epichlorhydrin hergestellt werden kann. So sollte z.B. ein Epichlorhydrin mit einem erhöhten Reinheitsgehalt, vorzugsweise von ca. 995 bis ca. 999,95 g/kg erhalten werden, insbesondere unter Verminderung des Chlorkohlenwasserstoffgehaltes des eingesetzten technischen Epichlorhydrins.

Erfindungsgemäß wurde festgestellt, daß diesen Zielen und Aufgaben ein Verfahren zur Herstellung von Reinst-Epichlorhydrin aus technische Verunreinigungen enthaltenden Epichlorhydrin gerecht wird, durch Destillation in einer Kolonne, die mit einer am Boden oder in der Nähe des Bodens der Kolonne angeordneten Verdampfungs- oder Erhitzungsvorrichtung ausgestattet ist.

Gemäß der Erfindung wird das technische Epichlorhydrin einer fraktionierten Destillation, vorzugsweise in einer Siebboden-, Glocken- und/oder Füllkörperkolonne, unterworfen, wobei das technische Epichlorhydrin in einem Abstand von der Verdampfungs- oder Erhitzungsvorrichtung (oder dem Verdampfungs- oder Erhitzungsbereich oder der -zone) eingeleitet wird, der größer als ein Zehntel, vorzugsweise größer als ein Viertel der gesamten Kolonne (Kolonnenlänge) ist und das Reinst-Epichlorhydrin wird in einem Abstand von der Verdampfungs- oder Erhitzungsvorrichtung (oder dem Verdampfungs- oder Erhitzungsbereich oder der Verdampfungszone) abgezogen, der größer als ein Drittel, vorzugsweise größer als die Hälfte der gesamten Kolonne ist.

Die leicht siedenden Bestandteile (Epichlorhydrin mit einem höheren Prozentgehalt an Verunreinigungen und/oder leicht siedende azeotrope Gemische) werden dabei vorzugsweise am Kopf der Kolonne und die schwersiedenden Bestandteile (Epichlorhydrin mit einem höheren Prozentgehalt an Verunreinigungen und/oder schwersiedende azeotrope Gemische mit Epichlorhydrin) vorzugsweise am Fuß der Kolonne kontinuierlich abgezogen.

Das erfindungsgemäße Verfahren wird bei einem Druck in der Kolonne von 0,2 bis 1,3 bar, vorzugsweise 0,5 bis 1 bar oder 1,05 bar, durchgeführt. Besonders vorteilhaft wird jedoch bei Normaldruck (geringer apparativer Aufwand) oder bei einem Unterdruck von 0,8 bis 0,98 bar (u.a. schonende Verdampfung des Epichlorhydrins) gearbeitet.

Der Mindestabstand der Einleitung oder Einleitungsstelle(n) für das technische (zu reinigende Epichlorhydrin) von der Verdampfungs- oder Erhitzungsvorrichtung (dem Verdampfungs- oder Erhitzungsbereich oder der Verdampfungszone) hängt u.a. von der Gesamtlänge der Kolonne, der Bestückung mit bestimmten Füllkörpern und/oder dem Mindestabstand der Siebböden ab und ist z.B. bei sehr langen Kolonnen oder starken oder dichten gefüllten Füllkörperkolonnen und dgl. größer als ein Zehntel der Gesamtlänge der Kolonne. Besonders zweckmäßig ist jedoch dieser Mindestabstand größer als ein Achtel oder ein Sechstel, vorzugsweise größer als ein Viertel, der Gesamtlänge der Kolonne.

Nach einer bevorzugten Ausführungsform des erfindungsgemäßen Verfahrens wird das technische Epichlorhydrin auf eine Temperatur unterhalb der Siedetemperatur vorgeheizt, bevor die Einleitung in die Kolonne erfolgt. Diese Vorheizung ist zusammen mit den vorgenannten Verfahrensmaßnahmen von besonderer Wichtigkeit für den zu erzielenden Reinheitsgrad des Reinst-Epichlorhydrins.

Der Temperaturunterschied zwischen der Vorheizung und der Betriebstemperatur in der Kolonne beträgt gemäß dem erfindungsgemäßen Verfahren höchstens 50 °C, vorzugsweise höchstens 10 °C. Ziel ist dabei, durch die Vorheizung das in die Kolonne einzuleitende technische Epichlorhydrin auf die gleiche oder annähernd gleiche Betriebstemperatur zu bringen wie sie in der Kolonne herrscht.

Das erfindungsgemäße Verfahren wird bevorzugt als kontinuierliches Verfahren durchgeführt, dergestalt, daß das vorgeheizte technische Epichlorhydrin kontinuierlich in die Kolonne in einem Abstand von der Verdampfungs- oder Erhitzungsvorrichtung eingeleitet wird, der größer als ein Zehntel, vorzugsweise größer als ein Viertel der gesamten Kolonnenlänge ist. Während man das Reinst-Epichlorhydrin in einem Abstand oberhalb der Einleitungsstelle abzieht, werden die Verunreinigungen am Kopf und Sumpf kontinuierlich abgezogen.

Die Pumpenleistung für die Zuleitung des technischen Epichlorhydrins wird gemäß der Erfindung so geregelt, daß die Zulaufmenge gleich oder annähernd gleich der Summe der Ablauf- oder Abzugsmengen ist.

Die Verdampfung und/oder Erhitzung des Epichlorhydrins in der Kolonne wird bevorzugt unter Verwendung eines Umlaufverdampfers durchgeführt. Nach einer bevorzugten Ausführungsform des erfindungsgemäßen Verfahrens wird zwischen der (bzw. den) Einleitungsstelle(n) des technischen Epichlorhydrins in die Kolonne und der (bzw. den) Abnahmestelle(n) für das Reinst-Epichlorhydrin ein Abstand eingehalten, der größer als ein Fünftel der Kolonnenlänge, vorzugsweise größer als ein Viertel der Kolonnenlänge, ist. Das Reinst-Epichlorhydrin wird dabei oberhalb der Einleitungsstelle(n) des technischen Epichlorhydrins abgezogen.

Die Erfindung betrifft weiterhin eine Vorrichtung zur Herstellung von Reinst-Epichlorhydrin aus technische Verunreinigungen enthaltendem Epichlorhydrin, bestehend aus mindestens einer Kolonne, die mit einer am Boden oder in der Nähe des Bodens der Kolonne angeordneten Verdampfungs- oder Erhitzungsvorrichtung ausgestattet ist, sowie mindestens eine Zuleitung und eine oder mehrere Ableitungsvorrichtungen aufweist. Gemäß der Erfindung ist bei der senkrechten Destillationskolonne die Abnahmestelle für das Reinst-Epichlorhydrin oberhalb der Einleitungsstelle des technischen Epichlorhydrins angeordnet. Unterhalb der Einleitungsstelle(n) des technischen Epichlorhydrins ist die Erhitzungs- oder Verdampfungsvorrichtung für die Kolonne, vorzugsweise für die Siebboden-, Glocken- und/oder Füllkörperkolonne, angeordnet.

Der Abstand der Einleitungsstelle(n) für das technische Epichlorhydrin von der Verdampfungs- oder Erhitzungsvorrichtung ist größer als ein Zehntel, vorzugsweise größer als ein Viertel, bezogen auf die gesamte Kolonnenlänge, während der Abstand der Abnahmestelle(n) für das Reinst-Epichlorhydrin von der Einleitungsstelle(n) des Epichlorhydrin größer als ein Fünftel, vorzugsweise größer als ein Viertel, der Kolonnenlänge ist. Nach einer besonders zweckmäßigen Ausführungsform wurde ein Abstand der Einleitungsstelle(n) für das technische Epichlorhydrin von der Verdampfungs- oder Erhitzungsvorrichtung eingehalten, der größer war als ein Sechstel, vorzugsweise größer als ein Viertel, bezogen auf die gesamte Kolonnenlänge.

Die Verdampfungs- oder Erhitzungsvorrichtung der Kolonne besteht bevorzugt aus einem Umlaufverdampfer.

Der Kolonne ist eine Vorheizvorrichtung für das einzuleitende technische Epichlorhydrin zugeordnet. Die Vorheizvorrichtung ist bevorzugt an oder in einem Einleitungsgefäß oder einer Einleitungsvorrichtung bzw. an oder in einem Vorratsgefäß oder einem ähnlichen Behälter angeordnet, der zur Aufnahme des einzuführenden technischen Epichlorhydrins dient und der vorzugsweise in seiner Einleitungsvorrichtung, vorzugsweise Einleitungsrohr und dgl., das bzw. die mit der Kolonne in Verbindung steht, mit Dosier- und/oder Verschlußvorrichtungen, Ventilen und dgl. versehen ist. Weiterhin sind nach einer Ausführungsform an dem Einleitungsgefäß oder an der Einleitungsvorrichtung Pumpen, Injiziervorrichtungen und dgl. angeordnet.

Die Erfindung betrifft weiterhin die Verwendung des Reinst-Epichlorhydrins, vorzugsweise hergestellt nach dem erfindungsgemäßen Verfahren zur Herstellung von Epoxidharzen für elektronische Bauteile, elektronische Bauelemente und Mikrochips.

Das so hergestellte Reinst-Epichlorhydrin, das im wesentlichen eine erhebliche Reduktion der Halogenkohlenwasserstoffe enthält bzw. im wesentlichen frei von Halogenkohlenwasserstoffen ist, wird in an sich bekannter Weise mit ein- oder mehrwertigen Phenolen, Carbonsäuren oder Aminen, vorzugsweise aromatischen Aminen, umgesetzt. Die erhaltenen Epoxidharze werden ohne oder nur unter Mitverwendung geringer Chlor- oder Halogenionen enthaltender Verarbeitungshilfsmittel zu elektronischen Bauteilen, Bauelementen oder Mikrochips umgeformt oder verarbeitet.

Für den kontinuierlichen betrieb bei der Herstellung des Reinst-Epichlorhydrins wird bevorzugt im Labormaßstab eine Kolbendosierpumpe bei der Einleitung des technischen Epichlorhydrins in die Kolonne verwendet. Die Dosierleistung wird über einen Regler, vorzugsweise Niveauregler in der Sumpfphase gesteuert. Bevorzugt ist der Kopfabzug mit einer Steuervorrichtung versehen, vorzugsweise einem magnetisch oder pneumatisch gesteuerten Flüssigkeitsteiler. Der Sumpf- und Produktabzug verfügt über an sich bekannte Pumpen, beispielsweise Kolben- oder Membrandosierpumpen. Als Sumpfheizer oder Umlaufverdampfer werden an sich bekannte Verdampfungs-, vorzugsweise Umlaufverdampfungsvorrichtungen, z.B. Quarzelektroheizstäbe oder Metalleinsatzheizvorrichtungen und dgl., gesteuert über Sonden im Sumpf und Regler, eingesetzt.

Ausführungsbeispiel

Auf Höhe des 8. Bodens (vom Fuß der Kolonne gerechnet) einer Siebbodenkolonne mit insgesamt 35 Böden wurden unter Normaldruck 2,2 l auf 114 °C vorgeheiztes technisches Epichlorhydrin mit einem Gehalt von 999,1 g/kg eingegeben. Am Kopf der Kolonne wurden stündlich 300 ml eines Gemisches aus Epichlorhydrin und leichtsiedenden Bestandteilen und am Sumpf stündlich 350 ml eines Gemisches aus Epichlorhydrin und schwersiedenden Bestandteilen entnommen.

Auf Höhe des 25. Bodens wurden bei 116 °C pro Stunde 1550 ml eines Reinst-Epichlorhydrins mit einem Gehalt von 999,7 g/kg entnommen.

## Ansprüche

1. Verfahren zur Herstellung von Reinst-Epichlorhydrin aus technische Verunreinigungen enthaltendem Epichlorhydrin durch Destillation in einer Kolonne, die mit einer am Boden oder in der Nähe des Bodens der Kolonne angeordneten Verdampfungs- oder Erhitzungsvorrichtung ausgestattet ist, dadurch gekennzeichnet, daß das technische Epichlorhydrin einer fraktionierten Destillation, vorzugsweise in einer Siebboden-, Glocken- und/oder Füllkörperkolonne, unterworfen wird, wobei das technische Epichlorhydrin in einem Abstand von der Verdampfungs- oder Erhitzungsvorrichtung eingeleitet wird, der
größer als 1/10, vorzugsweise
größer als 1/4,
der gesamten Kolonne beträgt und das Reinst-Epichlorhydrin in einem Abstand von der Verdampfungs- oder Erhitzungsvorrichtung abgezogen wird, der größer als ein Drittel, vorzugsweise größer als die Hälfte der gesamten Kolonne ist.

2. Verfahren nach Anspruch 1, dadurch gekennzeichnet, daß leichtsiedende Bestandteile (Verunreinigungen) und/oder leicht siedende azeotrope Gemische mit Epichlorhydrin am Kopf der Kolonne und schwersiedende Bestandteile (Verunreinigungen) und/oder schwersiedende azeotrope Gemische mit Epichlorhydrin am Fuß der Kolonne kontinuierlich abgezogen werden.

3. Verfahren nach Ansprüchen 1 und 2, dadurch gekennzeichnet, daß das technische Epichlorhydrin auf eine Temperatur unterhalb der Siedetemperatur vorgeheizt wird, bevor die Einleitung in die Kolonne erfolgt.

4. Verfahren nach einem oder mehreren der Ansprüche 1 bis 3, dadurch gekennzeichnet, daß der Temperaturunterschied zwischen der Vorheizung und der Betriebstemperatur in der Kolonne höchstens 50 °C, vorzugsweise
höchstens 10 °C,
beträgt.

5. Verfahren nach einem oder mehreren der Ansprüche 1 bis 4, dadurch gekennzeichnet, daß kontinuierlich vorgeheiztes technisches Epichlorhydrin in die Kolonne in einem Abstand von der Verdampfungs- oder Erhitzungsvorrichtung eingeleitet wird, der
größer als 1/10, vorzugsweise
größer als 1/4,
der gesamten Kolonne beträgt und kontinuierlich das Reinst-Epichlorhydrin oberhalb der Einleitungsstelle(n), während die Verunreinigungen in Kopf und Sumpf abgezogen werden.

6. Verfahren nach einem oder mehreren der Ansprüche 1 bis 5, dadurch gekennzeichnet, daß die Pumpenleistung für die Zuleitung des technischen Epichlorhydrins so geregelt wird, daß die Zulaufmenge gleich der Summe der Ablauf- oder Abzugsmengen entspricht.

7. Verfahren nach einem oder mehreren der Ansprüche 1 bis 6, dadurch gekennzeichnet, daß die Erhitzung des Epichlorhydrins in der Kolonne unter Verwendung eines Umlaufverdampfers erfolgt.

8. Verfahren nach einem oder mehreren der Ansprüche 1 bis 7, dadurch gekennzeichnet, daß zwischen der Einleitungsstelle des technischen Epichlorhydrins in die Kolonne und der Abnahmestelle für das Reinst-Epichlorhydrin ein Abstand eingehalten wird, der
größer als 1/5 der Kolonnenlänge, vorzugsweise
größer als 1/4 der Kolonnenlänge,
ist.

9. Verfahren nach einem oder mehreren der Ansprüche 1 bis 9, dadurch gekennzeichnet, daß das Reinst-Epichlorhydrin oberhalb der Einleitungsstelle abgezogen wird.

10. Verfahren nach einem oder mehreren der Ansprüche 1 bis 9, dadurch gekennzeichnet, daß die Destillation des Epichlorhydrins in der Kolonne bei einem Druck von
0,2 bis 1,05 bar, vorzugsweise
0,5 bis 1 bar,
durchgeführt wird.

11. Vorrichtung zur Herstellung von Reinst-Epichlorhydrin aus technische Verunreinigungen enthaltenden Epichlorhydrin, bestehend aus mindestens einer Kolonne, die mit einer am Boden oder in der Nähe des Bodens der Kolonne angeordneten Verdampfungs- oder Erhitzungsvorrichtung ausgestattet ist, sowie mindestens eine Zuleitung und eine oder mehrere Ableitungsvorrichtungen aufweist, dadurch gekennzeichnet, daß bei der senkrechten Destillationskolonne die Abnahmestelle für das Reinst-Epichlorhydrin oberhalb der Einleitungsstelle des technischen Epichlorhydrins angeordnet ist.

12. Vorrichtung nach Anspruch 11, dadurch gekennzeichnet, daß der Abstand der Einleitungsstelle von der Verdampfungs- oder Erhitzungsvorrichtung
größer als 1/10, vorzugsweise
größer als 1/4,
bezogen auf die gesamte Kolonnenlänge ist.

13. Vorrichtung nach Ansprüchen 11 und 12, dadurch gekennzeichnet, daß der Abstand der Abnahmestelle für das Reinst-Epichlorhydrin von der Einleitungsstelle des Epichlorhydrin
größer als 1/5, vorzugsweise
größer als 1/4,
der Kolonnenlänge beträgt.

14. Vorrichtung nach einem oder mehreren der Ansprüche 11 bis 13, dadurch gekennzeichnet, daß die Verdampfungs- oder Erhitzungsvorrichtung der Kolonne aus einem Umlaufverdampfer besteht.

15. Vorrichtung nach einem oder mehreren der Ansprüche 11 bis 14, dadurch gekennzeichnet, daß der Kolonne eine Vorheizvorrichtung für das einzuleitende technische Epichlorhydrin zugeordnet ist.

16. Verwendung des Reinst-Epichlorhydrins, vorzugsweise hergestellt nach einem oder mehreren der Ansprüche 1 bis 10, zur Herstellung von Epoxidharzen für elektronische Bauteile, elektronische Bauelemente und Mikrochips.

17. Verwendung des Verfahrens nach einem oder mehreren der Ansprüche 1 bis 10 zur Herstellung von Epichlorhydrin mit einem Epichlorhydringehalt von
größer als 998,0 g/kg, vorzugsweise
größer als 999,5 g/kg.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-B-1 251 299 (KNAPSACK AG) <br> * Insgesamt * <br> --- | 1-17 | C 07 D 301/32 <br> C 07 D 303/08 |
| X | CH-A- 373 366 (CONSORTIUM FÜR ELEKTROCHEMISCHE INDUSTIRE GmbH) <br> * Insgesamt * <br> ----- | 1-17 | |

**RECHERCHIERTE SACHGEBIETE (Int. Cl.4)**

C 07 D 301/00
C 07 D 303/00

Der vorliegende Recherchenbericht wurde für alle Patentansprüche erstellt

| Recherchenort | Abschlußdatum der Recherche | Prüfer |
|---|---|---|
| DEN HAAG | 04-07-1989 | ALLARD M.S. |